# FASCICULE DE BREVET EUROPEEN

(11) **EP 3 374 006 B1**
(45) Date de publication et mention de la délivrance du brevet: **20.04.2022**
(21) Numéro de dépôt: 16809488.6
(22) Date de dépôt: 10.11.2016
(51) Int. Cl.: A61M 5/20

(54) **AUTOINJECTEUR AYANT UNE FENÊTRE MEDIANE AYANT UNE EXTREMITE EN TRIANGLE**
AUTOINJEKTOR MIT EINEM MITTELFENSTER MIT EINEM DREIECKIGEN ENDE
AUTO-INJECTOR HAVING A MEDIAN WINDOW WITH ONE TRIANGULAR END

(30) Priorité: 13.11.2015 FR 1560879
(43) Date de publication de la demande: 19.09.2018
(73) Titulaire: Aptar France SAS, 27110 Le Neubourg (FR)
(72) Inventeur: FOUCAULT, Franck, 76650 Petit Couronne (FR); PINHEIRO, Philippe, 27400 Incarville (FR); SAUSSAYE, Anthony, 61300 Saint Sulpice Sur Risle (FR)
(74) Mandataire: CAPRI
(86) Numéro de dépôt international: PCT/FR2016/052926
(87) Numéro de publication internationale: WO 2017/081422

(56) Documents cités:
- EP-A1- 2 716 318
- WO-A1-2015/036346
- WO-A1-2015/155482
- US-A1- 2013 079 718

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique la pénétration de l'aiguille dans le corps du patient ainsi que l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois un certain nombre d'inconvénients.

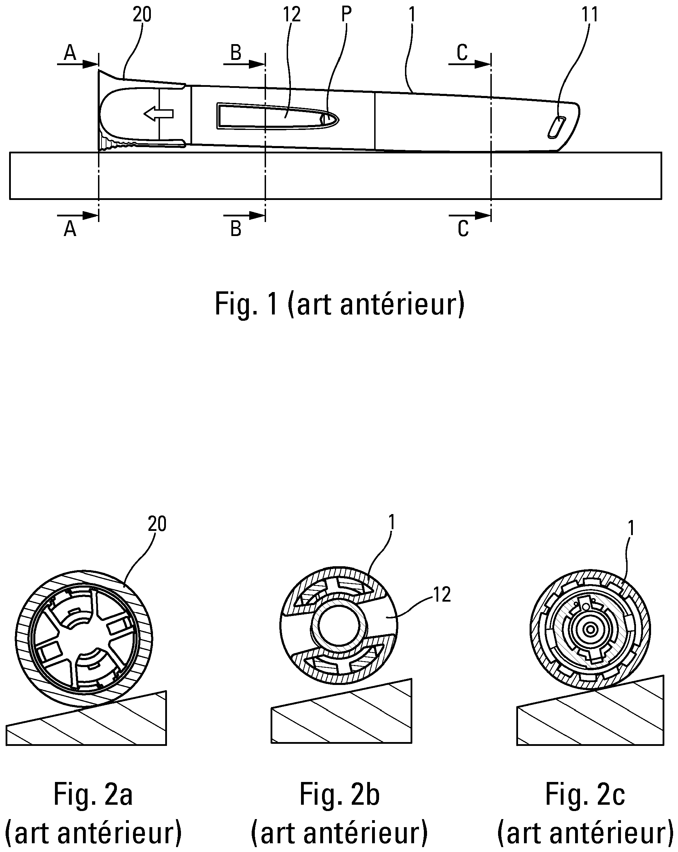

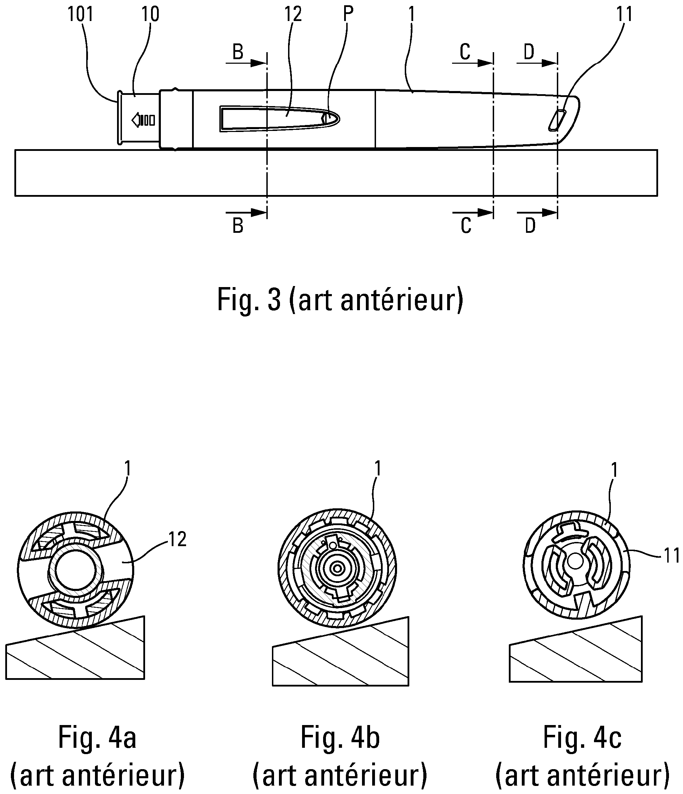

Ainsi, les autoinjecteurs existants sont souvent de forme cylindrique allongée. Ceci peut poser des problèmes lorsque l'autoinjecteur est posé à plat sur une surface inclinée, avec l'autoinjecteur risquant de se déplacer en roulant sur lui-même, avec un risque de perte ou d'endommagement du dispositif, par exemple s'il tombe par terre. Les figures 1 à 4 illustrent cet inconvénient. On constate sur les figures 1 et 2 que lorsque le capot amovible 20 est toujours en place, il y a deux points de contact entre le dispositif et la surface inclinée, à savoir au niveau dudit capot (figure 2a) et au niveau de la partie distale du corps (figure 2c), alors que la partie médiane est décollée de ladite surface inclinée (figure 2b). Du fait des profils circulaires au niveau desdits deux points de contact, l'autoinjecteur va rouler sur ladite surface inclinée. Les figures 3 et 4 montrent la situation lorsque le capot amovible 20 a été retiré. On constate alors qu'il n'y a plus que la partie médiane du corps en contact avec la surface inclinée (figure 4a), alors que les parties proximale et distale (figures 4b et 4c) sont décollées de ladite surface inclinée. Puisque le profil de ladite partie médiane est aussi circulaire, l'autoinjecteur va aussi rouler sur ladite surface inclinée. Ainsi, avec ou sans capot amovible, l'autoinjecteur des figures 1 à 4 ne restera pas immobile sur une surface inclinée.

Un autre inconvénient avec une telle forme allongée peut être qu'un utilisateur non avertit ou mal voyant risquerait de tenter une utilisation de l'autoinjecteur dans le mauvais sens, avec un risque de blessure par piqure par l'aiguille et un risque de perte de dose. Ainsi, l'autoinjecteur de la figure 3, s'il était utilisé dans le mauvais sens, risquerait de blesser l'utilisateur avec l'aiguille.

Les documents EP2716318, WO2015036346, US2013079718 et WO2015155482 décrivent des dispositifs de l'état de la technique.

La présente invention a pour but de fournir un autoinjecteur qui ne reproduit pas les inconvénients susmentionnés.

La présente invention a aussi pour but de fournir un autoinjecteur qui soit fiable d'utilisation, qui soit sûr et qui empêche tout risque de blessure, et qui soit simple et peu coûteux à fabriquer, à assembler et à utiliser.

La présente invention a donc pour objet un autoinjecteur tel que décrit dans la revendication 1. Des modes de réalisation avantageux sont décrits dans les revendications dépendantes.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
La figure 1 est une vue schématique de côté d'un autoinjecteur selon l'art antérieur, avant retrait du capuchon de protection,
La figure 2a est une vue schématique en coupe selon la ligne A-A de la figure 1, avec l'autoinjecteur disposé sur une surface inclinée,
La figure 2b est une vue schématique en coupe selon la ligne B-B de la figure 1, avec l'autoinjecteur disposé sur une surface inclinée,
La figure 2c est une vue schématique en coupe selon la ligne C-C de la figure 1, avec l'autoinjecteur disposé sur une surface inclinée,
La figure 3 est une vue schématique de côté de l'autoinjecteur de la figure 1, après retrait du capuchon de protection,
La figure 4a est une vue schématique en coupe selon la ligne B-B de la figure 3, avec l'autoinjecteur disposé sur une surface inclinée,
La figure 4b est une vue schématique en coupe selon la ligne C-C de la figure 3, avec l'autoinjecteur disposé sur une surface inclinée,
La figure 4c est une vue schématique en coupe selon la ligne D-D de la figure 3, avec l'autoinjecteur disposé sur une surface inclinée,
La figure 5 est une vue schématique de côté d'un autoinjecteur selon un mode de réalisation avantageux de la présente invention, avant retrait du capuchon de protection,
La figure 6a est une vue schématique en coupe selon la ligne A-A de la figure 5, avec l'autoinjecteur disposé sur une surface inclinée,
La figure 6b est une vue schématique en coupe selon la ligne B-B de la figure 5, avec l'autoinjecteur disposé sur une surface inclinée,
La figure 6c est une vue schématique en coupe selon la ligne C-C de la figure 5, avec l'autoinjecteur disposé sur une surface inclinée,
La figure 6d est une vue schématique en coupe selon la ligne D-D de la figure 5, avec l'autoinjecteur disposé sur une surface inclinée,
La figure 7 est une vue schématique de côté de l'autoinjecteur de la figure 5, après retrait du capuchon de protection,
La figure 8a est une vue schématique en coupe selon la ligne B-B de la figure 7, avec l'autoinjecteur disposé sur une surface inclinée,
La figure 8b est une vue schématique en coupe selon la ligne C-C de la figure 7, avec l'autoinjecteur disposé sur une surface inclinée,
La figure 8c est une vue schématique en coupe selon la ligne D-D de la figure 7, avec l'autoinjecteur disposé sur une surface inclinée,
La figure 9a est une vue en perspective d'un capot amovible selon une variante de réalisation,
La figure 9b est une vue similaire à celle de la figure 6a, avec le capot amovible de la figure 9a,
Les figures 10a à 10d sont des vues schématiques de coté de quatre variantes de réalisation,
Les figures 11a à 11d sont des vues schématiques de coté de quatre autres variantes de réalisation, et
Les figures 12a à 12d sont des vues schématiques de coté de quatre autres variantes de réalisation.

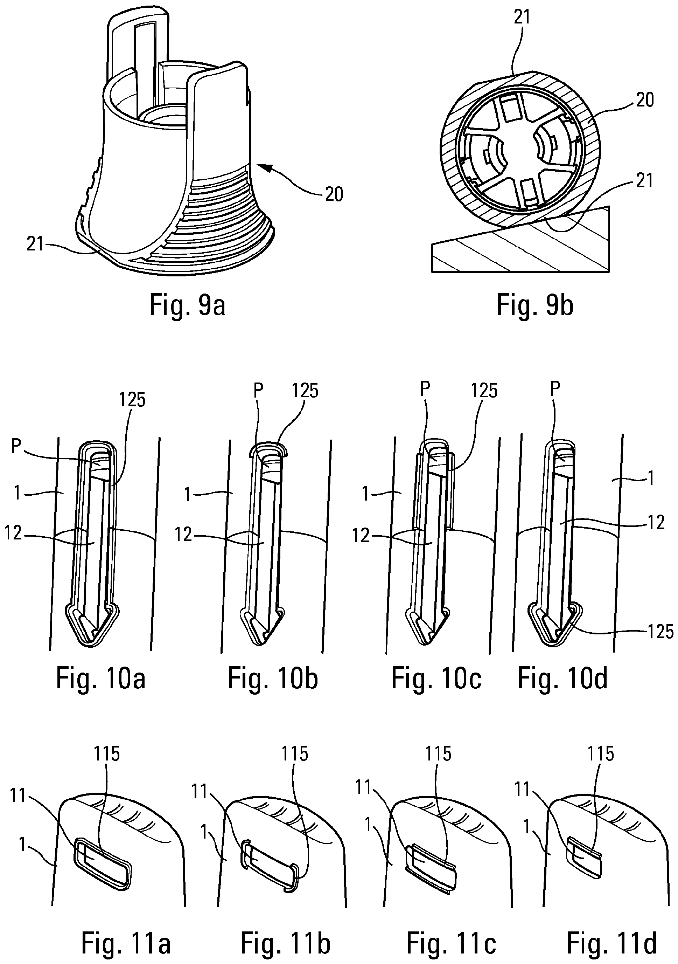

Dans la description ci-après, les termes "proximal" et "distal" se réfèrent à l'aiguille de l'autoinjecteur. Les termes "axial" et "radial" se réfèrent à l'axe central longitudinal de l'autoinjecteur. Les termes "supérieur" et "inférieur" se réfèrent à l'orientation des figures 11a à 11d.

L'autoinjecteur va être décrit ci-après en référence à des modes de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas tous les éléments constitutifs d'un autoinjecteur, mais seulement les éléments nécessaires au fonctionnement de la présente invention. Ainsi, les structures internes de l'autoinjecteur, qui n'interviennent pas dans la présente invention, ne sont pas représentés sur les dessins dans des buts de clarté, et ne seront décrits que brièvement ci-après. De plus, divers éléments et modules supplémentaires et/ou complémentaires pourraient être associés à l'autoinjecteur représenté sur les figures.

L'autoinjecteur représenté sur les figures comporte un corps 1 dans lequel coulisse axialement un manchon actionneur 10, dont l'extrémité inférieure 101 est destinée à venir en contact avec le corps du patient autour de la zone d'injection. Avantageusement, l'autoinjecteur peut comporter un corps inférieur, un corps intermédiaire et un corps supérieur qui sont assemblés pour former le corps 1 de l'autoinjecteur. Ci-après, le terme "corps" et la référence numérique "1" seront utilisés pour désigner ledit corps unitaire formé par l'assemblage dudit corps inférieur avec ledit corps intermédiaire et ledit corps supérieur. Il est à noter que le corps 1 pourrait être formé d'un nombre quelconques de parties de corps, par exemple deux.

Le corps 1 comporte une partie proximale, du coté de l'aiguille, sur laquelle est assemblé un capot amovible 20 que l'utilisateur doit retirer avant actionnement. Le corps 1 comporte aussi une partie distale, axialement opposée à ladite aiguille, pourvue avantageusement d'au moins une fenêtre distale 11, et une partie médiane, pourvue avantageusement d'au moins une fenêtre médiane 12. Lesdites parties proximale, médiane et distale du corps 1 sont avantageusement de forme générale environ cylindrique. De même, le capot amovible 20 a avantageusement une forme de révolution environ cylindrique. Dans cette variante, avec ou sans ledit capot amovible, l'autoinjecteur a une forme allongée environ cylindrique, qui serait susceptible de rouler lorsque disposé sur un plan incliné.

Un réservoir (non représenté) est inséré dans ledit autoinjecteur. Ce réservoir contient du produit fluide, et comporte un piston P et une aiguille. Le piston P est adapté à se déplacer dans ledit réservoir pour injecter le produit fluide à travers ladite aiguille. La présente description sera faite en référence à une seringue, qui peut être de tout type. Plus généralement, il est entendu que le terme "seringue" dans la présente description englobe tout type de réservoir associé à une aiguille. De préférence, le réservoir est une seringue pré-remplie. Ledit réservoir est visible à travers ladite au moins une fenêtre médiane 12, comme représenté sur les figures 1, 3, 10 et 12. Avantageusement, il y a deux fenêtres médianes 12 diamétralement opposées sur la partie médiane du corps 1. Ladite au moins une fenêtre médiane 12 permet de voir 100% du liquide contenu dans le réservoir avant utilisation. Elle permet aussi de voir le piston en fin d'injection. Elle peut aussi montrer le piston avant utilisation.

L'autoinjecteur comporte également un système d'injection automatique, comportant notamment une tige de piston (non représentée) adaptée à coopérer avec le piston P pour le déplacer dans le réservoir pour distribuer le produit fluide à travers l'aiguille. Cette tige de piston est classiquement sollicitée par un ressort d'injection (non représenté) vers sa position de distribution, et retenue avant actionnement dans sa position de repos par une serrure d'injection appropriée. Des exemples de serrures d'injection avantageuses sont notamment décrites dans les documents WO2013175148 et WO2015155484.

L'autoinjecteur peut aussi comporter un dispositif d'indication visuel, sonore et/ou tactile pour indiquer à l'utilisateur, notamment par un son audible, par une vibration et/ou par une indication visuelle et/ou tactile, qu'il peut retirer l'autoinjecteur du site d'injection. En particulier, le dispositif d'indication peut comporter un ou plusieurs élément(s) indicateur(s) qui donne(nt) d'une part une indication visuelle, par un affichage adéquat dans ladite au moins une fenêtre distale 11 du corps 1, et d'autre part une indication sonore et/ou tactile. Avantageusement, il y a deux fenêtres distales 11 diamétralement opposées sur la partie distale du corps 1.

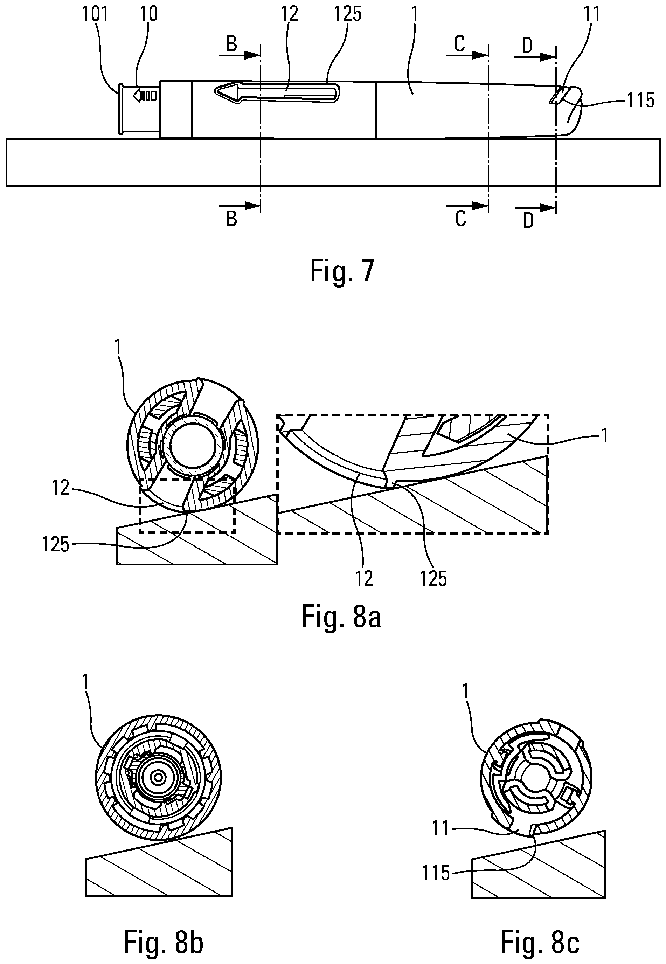

Le manchon actionneur 10 est sollicité vers ses positions projetées par un ressort (non représenté), qui peut être de type quelconque. Ce ressort coopère d'une part avec ledit manchon actionneur 10 et d'autre part avec une partie solidaire du corps 1. Avant actionnement, le manchon actionneur 10 est dans une première position projetée dans laquelle il entoure l'aiguille, comme représenté sur la figure 7. Lors de l'actionnement, le manchon actionneur 10 coulisse à l'intérieur du corps 1 vers une position d'actionnement, pour exposer l'aiguille et permettre le piquage puis l'injection du produit fluide. Après l'injection, lorsque l'utilisateur retire l'autoinjecteur du site d'injection, le manchon actionneur 10 revient dans une seconde position projetée de fin d'utilisation, dans laquelle il est à nouveau disposé autour de l'aiguille, pour éviter tout risque de blessure avec ladite aiguille. Il est à noter que les première et seconde positions projetées du manchon actionneur 10 peuvent être des positions différentes ou identiques.

Avant utilisation de l'autoinjecteur, l'aiguille de la seringue est avantageusement protégée par un capuchon, typiquement réalisé en élastomère, dans lequel l'extrémité de l'aiguille est piquée. Dans ce cas, le retrait dudit capot amovible 20 provoque avantageusement le retrait dudit capuchon de l'aiguille.

Lorsque l'utilisateur veut utiliser l'autoinjecteur, il prend le dispositif, par exemple au niveau du corps 1 et il appuie le manchon actionneur 10 contre la partie du corps où il veut réaliser l'injection. La pression exercée par l'utilisateur sur le manchon actionneur 10 provoque le coulissement de celui-ci vers l'intérieur du corps 1, ce qui découvre l'aiguille et donc son piquage en raison de la pression exercée par l'utilisateur sur l'autoinjecteur.

Lorsque le manchon actionneur 10 atteint sa position d'actionnement, qui est sa position d'extrémité à l'intérieur du corps 1, il provoque le déclenchement de la phase d'injection, pendant laquelle la tige de piston coulisse à l'intérieur de la seringue en poussant le piston P de celle-ci sous l'effet du ressort d'injection. Le produit est donc distribué.

A la fin de l'injection, le dispositif d'indication est actionné, éventuellement après un temps de retard prédéterminé, pour indiquer à l'utilisateur qu'il peut retirer l'autoinjecteur du site d'injection. Le manchon actionneur 10 est alors à nouveau déplacé hors du corps 1 vers sa seconde position projetée, sous l'effet de son ressort, avec un verrouillage dudit manchon actionneur 10, ce qui garantit une sécurité absolue pour l'utilisateur et évite tout risque de blessures avec l'aiguille après utilisation du dispositif.

Selon un aspect avantageux, l'autoinjecteur peut comporter au moins deux, avantageusement trois moyens anti-roulement empêchant ledit autoinjecteur de rouler sur une surface inclinée, avec ou sans capot amovible 20.

Ainsi, ladite partie médiane dudit corps 1 comporte des premiers moyens anti-roulement 125, formant au moins une zone non cylindrique médiane dans le profil externe de ladite partie médiane dudit corps 1. Ces premiers moyens anti-roulement 125 empêchent tout roulement après retrait du capot amovible 20, comme représenté sur les figures 7 et 8. On constate sur la figure 7 qu'en l'absence de capot amovible 20, les parties proximale et distale du corps 1 ne sont pas en contact avec la surface de support. Ceci est notamment illustré sur les figures 8b et 8c. C'est donc uniquement la partie médiane du corps 1 qui est en contact avec la surface, et lorsque cette surface est inclinée, les premiers moyens anti-roulement 125 empêchent un roulement de l'autoinjecteur, comme visible sur la figure 8a, dont la partie de droite est un agrandissement du détail de la partie de gauche.

De préférence, lesdits premiers moyens anti-roulement 125 sont formés par au moins une projection radialement saillante hors du profil externe environ cylindrique de ladite partie médiane dudit corps 1.

Lesdits premiers moyens anti-roulement 125 sont formés par au moins un bord radialement saillant de ladite au moins une fenêtre médiane 12. Les figures 10a à 10d illustrent quatre variantes de réalisation. Sur la figure 10a, ledit bord radialement saillant entoure complètement ladite fenêtre médiane 12. Sur la figure 10b, ledit bord radialement saillant entoure seulement la partie distale de ladite fenêtre médiane 12, alors que sur les figures 10c et 10d, ce sont respectivement les parties médiane et proximale de ladite fenêtre médiane 12 qui comportent respectivement un bord radialement saillant. Bien entendu d'autres variantes sont possibles. On prévoit deux fenêtres médianes 12 diamétralement opposées, comportant chacune des premiers moyens anti-roulement 125.

Comme visible sur la figure 8a, ledit bord radialement saillant forme un épaulement radial qui bloque toute rotation de l'autoinjecteur.

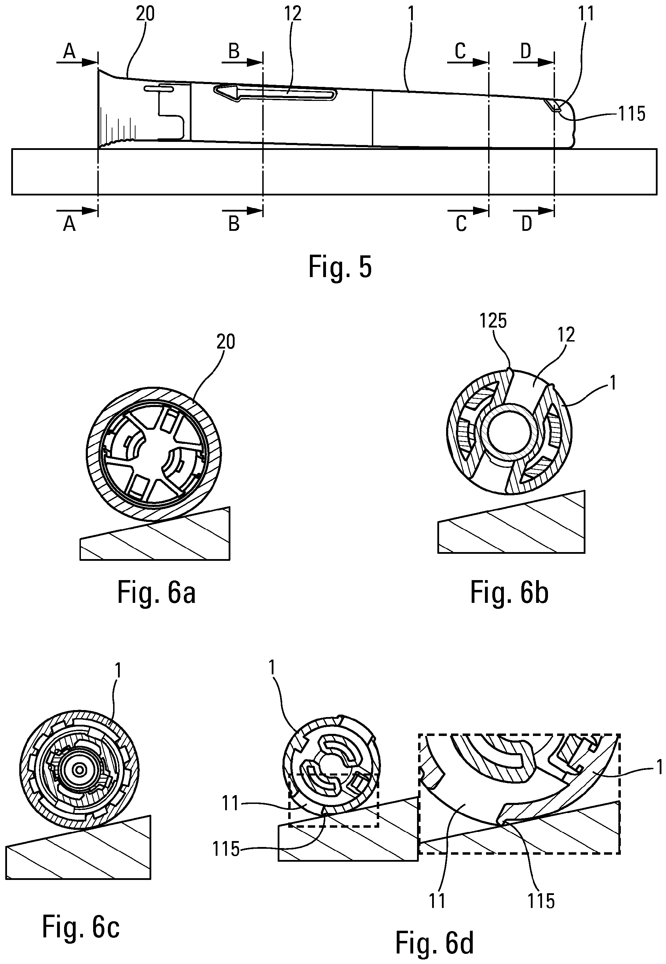

Comme visible sur les figures 5 et 6b, ladite partie médiane du corps 1, et notamment ladite fenêtre médiane 12, n'est pas en contact avec la surface de support lorsque le capot amovible 20 est en place. Les premiers moyens anti-roulement 125 ne sont donc pas efficaces dans ce cas. Il est donc nécessaire que l'autoinjecteur comporte d'autres moyens anti roulement qui agissent lorsque le capot amovible 20 est assemblé sur l'autoinjecteur.

Dans une première variante, ledit capot amovible 20 comporte des seconds moyens anti-roulement 21 formant au moins une zone non cylindrique proximale dans le profil externe dudit capot amovible 20. Avantageusement, ledit capot amovible 20 comporte une partie radialement externe de forme circulaire, lesdits seconds moyens anti-roulement 21 étant formés par au moins un méplat formé dans ladite partie radialement externe de forme circulaire. Ceci est représenté sur les figures 9a et 9b. De préférence, on prévoit deux méplats diamétralement opposés sur ledit capot amovible 20. Bien entendu d'autres variantes sont possibles, par exemple des projections radiales à la place des méplats.

Dans une seconde variante, ladite partie distale dudit corps 1 comporte des troisièmes moyens anti-roulement 115 formant au moins une zone non cylindrique distale dans le profil externe de ladite partie distale dudit corps 1.

De préférence, lesdits troisièmes moyens anti-roulement 115 sont formés par au moins une projection radialement saillante hors du profil externe environ cylindrique de ladite partie distale dudit corps 1.

Avantageusement, lesdits troisièmes moyens anti-roulement 115 sont formés par au moins un bord radialement saillant de ladite au moins une fenêtre distale 11. Les figures 11a à 11d illustrent quatre variantes de réalisation. Sur la figure 11a, ledit bord radialement saillant entoure complètement ladite fenêtre distale 11. Sur la figure 11b, lesdits bords radialement saillants sont formés seulement sur les cotés latéraux de ladite fenêtre distale 11, alors que sur les figures 11c, ce sont les cotés supérieur et inférieur de ladite fenêtre distale 11 qui comportent chacun un bord radialement saillant. Enfin, la figure 11d montre un bord radialement saillant formé seulement sur le coté supérieur de la fenêtre distale 11. Bien entendu d'autres variantes sont possibles. Avantageusement, on prévoit deux fenêtres distales 11 diamétralement opposées, comportant chacune des troisièmes moyens anti-roulement 115.

Comme visible sur la figure 6d, ledit bord radialement saillant forme un épaulement radial qui bloque toute rotation de l'autoinjecteur.

Bien entendu, on peut combiner les deux variantes ci-dessus.

Selon un autre aspect avantageux, ladite au moins une fenêtre médiane 12 a une forme extérieure en forme de flèche pointant vers la partie proximale dudit corps 1. Cette mise en œuvre a pour but d'éviter une utilisation de l'autoinjecteur dans le mauvais sens. La forme de flèche indique clairement à l'utilisateur dans quel sens il faut actionner le dispositif.

Avantageusement, cette forme de flèche est aussi facilement détectable par les mal voyants, notamment si ladite fenêtre médiane 12 comporte un bord radialement saillant, comme décrit précédemment.

Selon l'invention, la partie proximale 121 de la fenêtre médiane 12 comporte une partie d'extrémité en triangle, formant pointe de la flèche, la base de ladite partie d'extrémité en triangle, opposée à ladite pointe, formant la partie de plus grande largeur de ladite fenêtre médiane. Cette mise en œuvre permet de bien marquer la forme de flèche au niveau de la partie proximale de la fenêtre médiane 12.

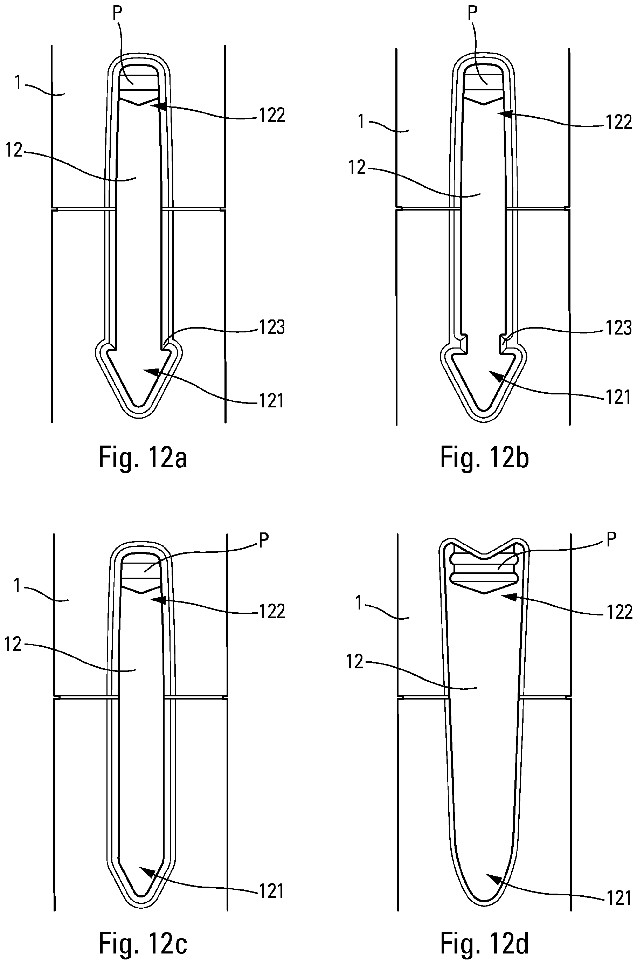

Les figures 12a à 12d illustrent quatre variantes de réalisation.

Sur les variantes des figures 12a et 12b, ladite partie proximale 121 en triangle, formant pointe de la flèche, est reliée au reste de la fenêtre par un épaulement 123. La figure 12b comporte un épaulement 123 plus marqué.

La figure 12c comporte aussi une partie d'extrémité proximale 121 en triangle, mais sans épaulement 123. Néanmoins, on voit bien que la base élargie de la pointe forme la partie la plus large de la fenêtre médiane 12.

Enfin la figure 12d comporte une partie proximale 121 de fenêtre plus arrondie, mais pointant toujours vers la partie proximale du corps 1. Cette variante de la partie proximale 121 n'est pas couverte par la présente invention puisque la partie la plus large de la fenêtre médiane 12 est située à sa partie distale 122. Cette variante de la figure 12d est toutefois intéressante car elle montre la partie distale 122 de la fenêtre 12 qui comporte un profil en forme de pointe orienté aussi vers la partie proximale du corps 1, ce qui renforce l'impression visuelle d'une fenêtre médiane 12 orientée vers l'aiguille. Bien entendu, cette partie distale 122 de la figure 12d pourrait être associée aux autres variantes de l'invention des figures 12a à 12c.

Bien entendu d'autres variantes sont possibles.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à des modes de réalisation avantageux, il est entendu que l'homme du métier peut y apporter diverses modifications sans sortir du cadre de la présente invention tel que défini par les revendications annexées.

## Revendications

1. Autoinjecteur comportant un corps (1) adapté à recevoir un réservoir contenant du produit fluide et comportant un piston (P) et une aiguille, tel qu'une seringue pré-remplie, ledit corps (1) comportant une partie distale axialement opposée à l'aiguille, une partie médiane et une partie proximale du côté de l'aiguille, ladite partie médiane dudit corps (1) comportant au moins une fenêtre médiane (12) montrant avant utilisation 100% du produit fluide contenu dans ledit réservoir et montrant après injection ledit piston (P), ladite au moins une fenêtre médiane (12) ayant une forme extérieure en forme de flèche pointant vers la partie proximale dudit corps (1), la partie proximale (121) de ladite au moins une fenêtre médiane (12) comportant une partie d'extrémité en triangle, formant pointe de la flèche, la base de ladite partie d'extrémité en triangle, opposée à ladite pointe, formant la partie de plus grande largeur de ladite fenêtre médiane (12), pour ainsi marquer la forme de flèche au niveau de ladite partie proximale de ladite fenêtre médiane (12), **caractérisé en ce que** des premiers moyens anti-roulement (125) sont formés par au moins un bord radialement saillant de ladite au moins une fenêtre médiane (12), ladite partie médiane dudit corps (1) comportant deux fenêtres médianes (12) diamétralement opposées comportant chacune lesdits premiers moyens anti-roulement (125)..

2. Autoinjecteur selon la revendication 1, dans lequel ladite partie d'extrémité en triangle est relié au reste de ladite fenêtre médiane (12) par un épaulement (123).

3. Autoinjecteur selon l'une quelconque des revendications précédentes, dans lequel la partie distale (122) de ladite au moins une fenêtre médiane (12) comporte un profil en forme de pointe orienté vers ladite partie proximale du corps (1).

4. Autoinjecteur selon l'une quelconque des revendications précédentes, dans lequel ledit au moins un bord radialement saillant entoure au moins partiellement ladite au moins une fenêtre médiane (12).

5. Autoinjecteur selon l'une quelconque des revendications précédentes, dans lequel un capot amovible (20) est fixé à ladite partie proximale dudit corps (1), avant utilisation de l'autoinjecteur.

6. Autoinjecteur selon l'une quelconque des revendications précédentes, dans lequel ledit autoinjecteur comporte un manchon actionneur (10) déplaçable par rapport audit corps (1) entre des positions projetées, dans lesquelles ledit manchon actionneur (10) fait au moins partiellement saillie hors dudit corps (1), et une position d'actionnement, dans laquelle ledit manchon actionneur (10) est axialement déplacé vers l'intérieur dudit corps (1), ledit manchon actionneur (10) étant dans une première position projetée avant actionnement de l'autoinjecteur et dans une seconde position projetée après actionnement de l'autoinjecteur, ledit manchon actionneur (10) étant sollicité vers lesdites positions projetées par un ressort.

7. Autoinjecteur selon la revendication 6, dans lequel ledit manchon actionneur (10) comporte une extrémité de contact (101) destinée à venir en contact avec la zone d'injection.

## Patentansprüche

1. Autoinjektor, der einen Körper (1) umfasst, der dafür ausgelegt ist, einen Vorratsbehälter aufzunehmen, der ein Fluidprodukt enthält und einen Kolben (P) sowie eine Nadel aufweist, etwa eine im Voraus befüllte Spritze, wobei der Körper (1) einen distalen Abschnitt axial gegenüber der Nadel, einen mittleren Abschnitt und einen proximalen Abschnitt auf Seiten der Nadel umfasst, wobei der mittlere Abschnitt des Körpers (1) wenigstens ein mittleres Fenster (12) aufweist, das vor der Verwendung 100 % des in dem Vorratsbehälter enthaltenen Fluidprodukts zeigt und nach der Injektion den Kolben (P) zeigt, wobei das wenigstens eine mittlere Fenster (12) eine äußere Form in Pfeilform, die zu dem proximalen Abschnitt des Körpers (1) zeigt, besitzt, wobei der proximale Abschnitt (121) des wenigstens einen mittleren Fensters (12) einen dreieckigen Endabschnitt aufweist, der die Pfeilspitze bildet, während die Basis des dreieckigen Endabschnitts gegenüber der Spitze den Abschnitt mit größerer Länge des mittleren Fensters (12) bildet, um so die Pfeilform auf Höhe des proximalen Abschnitts des mittleren Fensters (12) zu markieren, **dadurch gekennzeichnet, dass** durch wenigstens eine radiale Kante, die von dem wenigstens einen mittleren Fenster (12) vorsteht, erste Wälzverhinderungsmittel (125) gebildet werden, wobei der mittlere Abschnitt des Körpers (1) zwei diametral gegenüberliegende mittlere Fenster (12) aufweist, die jeweils die ersten Wälzverhinderungsmittel (125) aufweisen.

2. Autoinjektor nach Anspruch 1, wobei der dreieckige Endabschnitt mit dem restlichen mittleren Fenster (12) durch eine Schulter (123) verbunden ist.

3. Autoinjektor nach einem der vorhergehenden Ansprüche, wobei der distale Abschnitt (122) des wenigstens einen mittleren Fensters (12) ein Profil in Form einer Spitze besitzt, die zu dem proximalen Abschnitt des Körpers (1) zeigt.

4. Autoinjektor nach einem der vorhergehenden Ansprüche, wobei die wenigstens eine radial vorstehende Kante das wenigstens eine mittlere Fenster (12) wenigstens teilweise umgibt.

5. Autoinjektor nach einem der vorhergehenden Ansprüche, wobei an dem proximalen Abschnitt des Körpers (1) vor der Verwendung des Autoinjektors eine abnehmbare Kappe (20) befestigt ist.

6. Autoinjektor nach einem der vorhergehenden Ansprüche, wobei der Autoinjektor eine Betätigungshülse (10) aufweist, die in Bezug auf den Körper (1) zwischen vorstehenden Positionen, in denen die Betätigungshülse (10) wenigstens teilweise von dem Körper (1) vorsteht, und einer Betätigungsposition, in der die Betätigungshülse (10) axial in den Körper (1) verlagert ist, verlagerbar ist, wobei die Betätigungshülse (10) vor der Betätigung des Autoinjektors in einer ersten vorstehenden Position ist und nach der Betätigung des Autoinjektors in einer zweiten vorstehenden Position ist, wobei die Betätigungshülse (10) durch eine Feder in die vorstehenden Positionen vorbelastet ist.

7. Autoinjektor nach Anspruch 6, wobei die Betätigungshülse (10) ein Kontaktende (101) aufweist, das dazu bestimmt ist, mit der Injektionszone in Kontakt zu gelangen.

## Claims

1. An autoinjector comprising a body (1) that is adapted to receive a reservoir containing fluid and including a piston (P) and a needle, such as a pre-filled syringe, said body (1) comprising a distal portion axially opposite from said needle, a middle portion, and a proximal portion on the needle side, said middle portion of said body (1) including at least one middle window (12) showing, before use, 100% of the fluid contained in said reservoir, and showing, after injection, said piston (P), said at least one middle window (12) having an external shape in the shape of an arrow that points towards the proximal portion of said body (1), the proximal portion (121) of said at least one middle window (12) including a triangular end portion, forming the point of the arrow, the base of said triangular end portion, remote from said point, forming the widest portion of said middle window (12), so as to emphasize the arrow shape of the proximal portion of the middle window (12), **characterized in that** first anti rolling means (125) are formed by at least one radically projecting edge of said at least one middle window (12), said middle portion of said body (1) including two diametrically opposite middle windows (12), each including said first anti rolling means (125).

2. An autoinjector according to claim 1, wherein said triangular end portion is connected to the remainder of said middle window (12) via a shoulder (123).

3. An autoinjector according to either preceding claim, wherein the distal portion (122) of said at least one middle window (12) includes a profile in the shape of a point that points towards said proximal portion of the body (1).

4. An autoinjector according to either preceding claim, wherein said at least one radially-projecting edge surrounds said at least one middle window (12) at least in part.

5. An autoinjector according to any preceding claim, wherein a removable cap (20) is fastened to said proximal portion of said body (1) before the autoinjector is used.

6. An autoinjector according to any preceding claim, wherein said autoinjector includes an actuator sleeve (10) that is movable relative to said body (1) between projecting positions in which said actuator sleeve (10) projects out from said body (1) at least in part, and an actuated position in which said actuator sleeve (10) is moved axially into said body (1), said actuator sleeve (10) being in a first projecting position before actuation of the autoinjector, and in a second projecting position after actuation of the autoinjector, said actuator sleeve (10) being urged towards said projecting positions by a spring.

7. An autoinjector according to claim 6, wherein said actuator sleeve (10) includes a contact end (101) for coming into contact with the injection zone.
